# EUROPEAN PATENT APPLICATION

(11) **EP 3 332 719 A1**
(43) Date of publication of application: **13.06.2018**
(21) Application number: 16833026.4
(22) Date of filing: 02.08.2016
(51) Int. Cl.: A61B 17/32, A61B 17/295

(54) **FORCEPS-TYPE TREATMENT TOOL**

(30) Priority: 04.08.2015 JP 2015154114
(71) Applicant: Olympus Corporation, Hachioji-shi Tokyo 192-8507 (JP)
(72) Inventor: IDE, Katsushi, Hachioji-shi Tokyo 192-8507 (JP); KAGA, Tomoyuki, Hachioji-shi Tokyo 192-8507 (JP)
(74) Representative: Schicker, Silvia
(86) International application number: PCT/JP2016/072619
(87) International publication number: WO 2017/022747

(57) **Abstract**

A forceps-type treatment instrument includes an end effector including a pair of clamps. In one of the clamps, a swinger is extended along an extending axis and swings relative to a supporting section around a swing axis extended along a direction intersecting with the extending axis and intersecting with an open and close direction of the clamp. A regulating section regulates movement of the swinger in the direction along the swing axis in at least two portions separated from each other in a direction along the extending axis, and thereby prevents rotation of the swinger around a rotation axis along the open and close direction.

## Description

### Technical Field

The present invention relates to a forceps-type treatment instrument provided with an end effector in which a space between a pair of clamps is openable and closable.

### Background Art

U.S. Patent Application Publication No. 2013/303949 discloses a forceps-type treatment instrument provided with an end effector in which a space between a pair of clamps is openable and closable. In this forceps-type treatment instrument, a first clamp as one of the clamps includes a supporting section and a swinger swingable around a swing axis with respect to the supporting section. The swinger is extended along an extending axis from a proximal portion to a distal portion, and the swing axis is extended along a direction intersecting with the extending axis of the swinger and intersecting with an open and close direction of the first clamp. Furthermore, the forceps-type treatment instrument is provided with a vibration transmission member capable of transmitting ultrasonic vibration from a proximal side to a distal side, and the distal portion of the vibration transmitting member (projecting part from a sheath) forms a second clamp as the other clamp. The swinger of the first clamp includes a pad made of resin and a holding member made of metal. By closing the space between the clamps, the pad can abut on the second clamp. In a state where the pad abuts on the second clamp, the holding member is separated from the second clamp.

With a forceps-type treatment instrument in which the first clamp is provided with the supporting section and the swinger as disclosed in U.S. Patent Application Publication No. 2013/303949, since the swinger is swingably coupled to the supporting section, there is a possibility that a part of the swinger moves with respect to the supporting section in the direction along the swing axis. If a part of the swinger moves in the direction along the swing axis, the swinger rotates (rattles) with respect to the supporting section around the rotation axis that is substantially parallel to the open and close direction of the first clamp, and the angle of the extending axis of the swing axis with respect to the direction along the swing axis changes. As a result, the position of the swinger with respect to the second clamp in the width direction of the end effector changes, and for example, the holding member made of metal may come in contact with the second clamp. When the holding member comes into contact with the second clamp vibrating by ultrasonic vibration, the second clamp likely gets damaged.

### Summary of Invention

The present invention was designed to address the above problems, and the purpose of the present invention is to provide a forceps-type treatment instrument in which, in clamp provided with a swinger swingable to a supporting section, rotation (rattle) of a swinger around a rotation axis substantially parallel to an open and close direction is appropriately prevented.

To solve the above mentioned problems, according to one aspect of the invention, a forceps-type treatment instrument, including: an end effector including a first clamp, and a second clamp that is openable and closable relative to the first clamp; a supporting section provided in the first clamp; a swinger provided in the first clamp to be opposed to the second clamp, and extended along an extending axis from a distal portion to a proximal portion, the swinger swinging with respect to the supporting section around a swing axis, which is extended along a direction intersecting with the extending axis and intersecting with an open and close direction of the first clamp, the swinger swinging in a swinging range between a position where the distal portion comes closest to the second clamp and a position where the proximal portion comes closest to the second clamp; and a regulating section regulating movement of the swinger in the direction along the swing axis in at least two portions separated from each other in a direction along the extending axis, and thereby preventing rotation of the swinger around a rotation axis along the open and close direction of the first clamp, in a state where the swinger is located at any position in the swinging range.

### Brief Description of Drawings

FIG. 1 is a schematic view showing a treatment system using a forceps-type treatment instrument according to a first embodiment;
FIG. 2 is a schematic view showing a distal portion of the forceps-type treatment instrument according to the first embodiment in a state where a swinger is located at a neutral position, viewed from one side in a width direction of an end effector;
FIG. 3 is a cross-sectional view taken along X1-X1 in FIG. 2;
FIG. 4 is a cross-sectional view taken along X2-X2 in FIG. 2;
FIG. 5 is a schematic view showing the swinger according to the first embodiment, viewed from one side in a direction along a swing axis;
FIG. 6 is a schematic view showing the swinger according to the first embodiment, viewed from a back surface side in an open and close direction of the first clamp;
FIG. 7 is a cross-sectional view taken along X3-X3 in FIG. 2;
FIG. 8 is a cross-sectional view taken along X4-X4 and X6-X6 in FIG. 2;
FIG. 9 is a cross-sectional view taken along X5-X5 in FIG. 2;
FIG. 10 is a cross-sectional view taken along X7-X7 in FIG. 2;
FIG. 11 is schematic view showing the distal portion of the forceps-type treatment instrument according to the first embodiment in a state where the swinger is positioned where a proximal portion of the swinger comes closest to a second clamp, viewed from one side in the width direction of the end effector;
FIG. 12 is schematic view showing the distal portion of the forceps-type treatment instrument according to the first embodiment in a state where the swinger is positioned where a distal portion of the swinger comes closest to a second clamp, viewed from one side in the width direction of the end effector;
FIG. 13 is a cross-sectional view taken along X8-X8 in FIG. 11 and X12-X12 in FIG. 12;
FIG. 14 is a cross-sectional view taken along X9-X9 in FIG. 11 and X11-X11 in FIG. 12;
FIG. 15 is a cross-sectional view taken along X10-X10 in FIG. 11;
FIG. 16 is a cross-sectional view taken along X13-X13 in FIG. 12;
FIG. 17 is a schematic view showing the first clamp according to a first modification, viewed from one side in the width direction of the end effector;
FIG. 18 is a cross-sectional view taken along X14-X14 in FIG. 17;
FIG. 19 is a schematic view showing the first clamp according to a second modification, viewed from one side in the width direction of the end effector;
FIG. 20 is a schematic view showing the swinger according to the second modification, viewed from the back surface side in the open and close direction of the first clamp;
FIG. 21 is a cross-sectional view taken along X15-X15 in FIG. 19;
FIG. 22 is a schematic view showing the distal portion of the forceps-type treatment instrument according to a third modification, viewed from one side in the width direction of the end effector; and
FIG. 23 is a cross-sectional view taken along X16-X16 in FIG. 22.

### Description of Embodiments

### (First Embodiment)

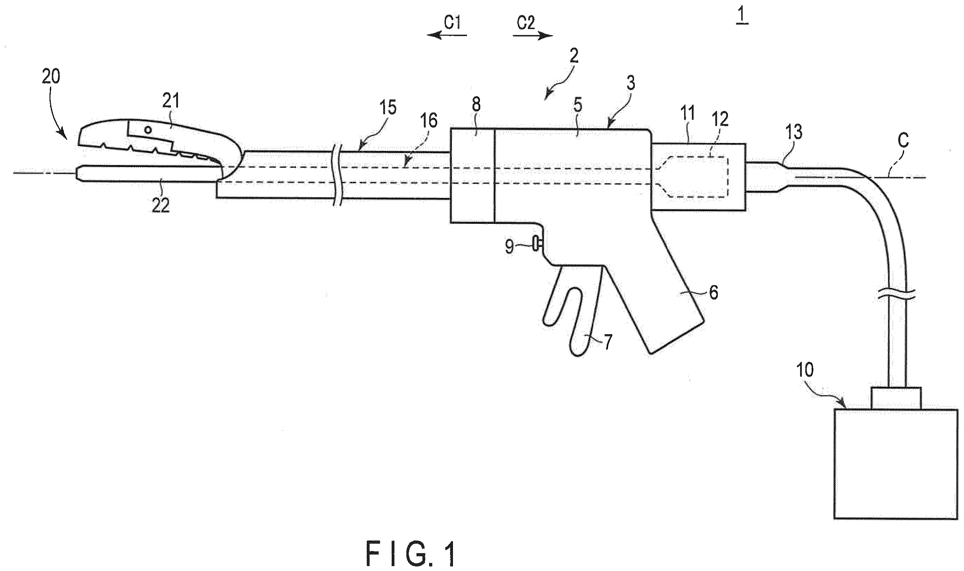

The first embodiment of the present invention will be described with reference to FIGS. 1 to 16.

FIG. 1 is a view showing a treatment system (ultrasonic treatment system) 1 using a forceps-type treatment instrument (ultrasonic treatment instrument) 2 of the present embodiment. As shown in FIG. 1, the forceps-type treatment instrument 2 has a longitudinal axis C. One side of the direction along the longitudinal axial C direction is a distal side (the side indicated by the arrow C1 in FIG. 1), and a side opposite to the distal side is a proximal side (the side indicated by the arrow C2 in FIG. 1). In the present embodiment, the forceps-type treatment instrument 2 uses ultrasonic vibration as treatment energy to treat a treated target.

The forceps-type treatment instrument 2 includes a holdable housing 3. The housing 3 includes a housing main body 5 extended along the longitudinal axis C, and a grip (stationary handle) 6 extended from the housing main body 5 toward a direction intersecting with the longitudinal axis C. A handle (movable handle) 7 is rotatably attached to the housing 3, and when the handle 7 is rotated with respect to the housing 3, the handle 7 is opened and closed with respect to the grip 6. A rotation knob (rotation operation section) 8 is coupled to the housing main body 5 of the housing 3 from the distal side. The rotation knob 8 is rotatable around the longitudinal axis C with respect to the housing 3. An operation button 9 as an operation input section for inputting energy operation is attached to the housing 3.

A transducer case 11 is coupled to the housing main body 5 from the proximal side. A vibration generator 12 is provided inside the transducer case 11. The vibration generator 12 is attached to the transducer case 11 and includes a piezoelectric element (not shown). One end of a cable 13 is connected to the transducer case 11. The other end of the cable 13 is detachably connected to an energy control device 10. The energy control device 10 includes a conversion circuit that converts DC electric power or AC electric power (e.g., electric power from a battery or an outlet) into electrical energy (vibration generation electrical energy), a processor including a central processing unit (CPU) or application specific integrated circuit (ASIC), and a storage medium such as a memory. The energy control device 10 is electrically connected to a switch (not shown) provided inside the housing 3, and detects an operation input performed by the operation button 9 based on the open/close state of the switch. The energy control device 10 outputs electrical energy based on the detection of the operation input by the operation button 9. The operation input section is not limited to the operation button 9 and may be, for example, a foot switch separate from the forceps-type treatment instrument 2.

A sheath 15 is fixed to the rotation knob 8 while being inserted inside the rotation knob 8 from the distal side. The sheath 15 is extended along the longitudinal axis C. A vibration transmission member (probe) 16 is extended from the inside of the housing main body 5 through the inside of the sheath 15 toward the distal side. The longitudinal axis C is extended through the inside of the vibration transmission member 16. A first clamp (jaw) 21 is rotatably attached to the distal portion of the sheath 15. A second clamp (probe treatment section) 22 is formed in the distal portion of the vibration transmission member 16, and the second clamp 22 projects from the distal end of the sheath 15 to the distal side. In the present embodiment, a pair of clamps (clamp portions) 21 and 22 form an end effector 20 that treats a treated target. By rotating the rotation knob 8, the vibration generator 12, the sheath 15, the vibration transmission member 16 (including the second clamp 22), and the first clamp 21 rotate together around the longitudinal axis C.

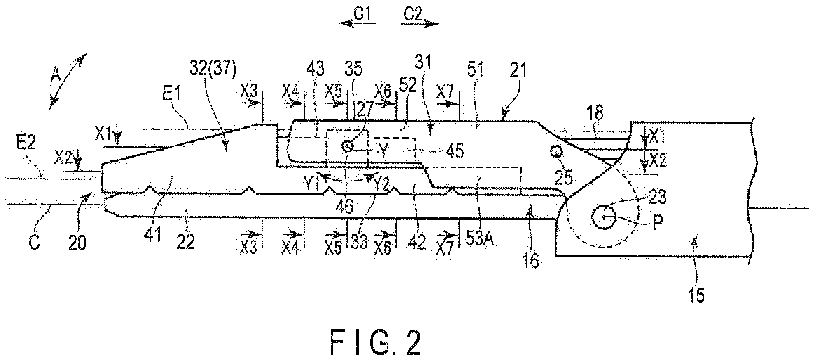

FIG. 2 is a view showing the distal portion of the forceps-type treatment instrument 2 including the end effector 20, which is viewed from one side in a width direction of the end effector 20. As shown in FIG. 2, the first clamp 21 includes a supporting section (supporting member) 31 attached to the distal portion of the sheath 15 through a supporting pin 23. The supporting section 31 is extended along an extending axis (first extending axis) E1 from the distal portion to the proximal portion and rotatable with respect to the sheath 15 around a rotation axis P formed by the supporting pin 23. The rotation axis P is extended substantially parallel with respect to the width direction of the end effector 20. A movable body 18 is extended along the longitudinal axis C inside the sheath 15. A distal end of the movable body 18 is connected to the supporting section 31 through a connection pin 25. The movable body 18 moves along the longitudinal axis C by opening or closing the handle 7 with respect to the grip 6. Thus, the first clamp 21 including the supporting section 31 rotates around the rotation axis P with respect to the sheath 15, and a space between the clamps 21 and 22 is opened or closed (the clamps 21 and 22 are relatively opened or closed).

In the first clamp 21, a swinger 32 is attached to the supporting section 31 through a supporting pin 27. The swinger 32 is extended along an extending axis (second extending axis) E2 from the distal portion to the proximal portion, and is swingable with respect to the supporting section 31 around a swing axis Y formed by the supporting pin 27. The swing axis Y is extended along a direction intersecting with (substantially perpendicular to) the extending axis E2 of the swinger 32 (extending axis E1 of the supporting section 31) and intersecting with (substantially perpendicular to) the open and close direction of the first clamp 21 (the direction indicated by the arrow A in FIG. 2). In the present embodiment, the swing axis Y is extended substantially parallel with respect to the width direction of the end effector 20. The swinger 32 is opposed to the second clamp 22. The swinger 32 forms an opposed surface (grasping surface) 33 opposed to the second clamp 22. The supporting section 31 and the swinger 32 form a back surface 35 that faces an opposite side of the opposed surface 33.

The swinger 32 swings around the swing axis Y, with a position where the extending axis E2 of the swinger 32 is substantially parallel to the extending axis E1 of the supporting section 31 (the position shown in FIG. 2) as a neutral position. When the swinger 32 swings to one side (the side indicated by the arrow Y1 in FIG. 2) in the swing directions from the neutral position, the distal portion of the swinger 32 (the portion at the distal side relative to the swing axis Y in the swinger 32) goes away from the second clamp 22, and the proximal portion of the swinger 32 (the portion at the proximal side relative to the swing axis Y in the swinger 32) comes close to the second clamp 22. On the other hand, when the swinger 32 swings to the other side (the side indicated by the arrow Y2 in FIG. 2) in the swing directions from the neutral position, the distal portion of the swinger 32 comes closer to the second clamp 22, and the proximal portion of the swinger 32 goes away from the second clamp 22.

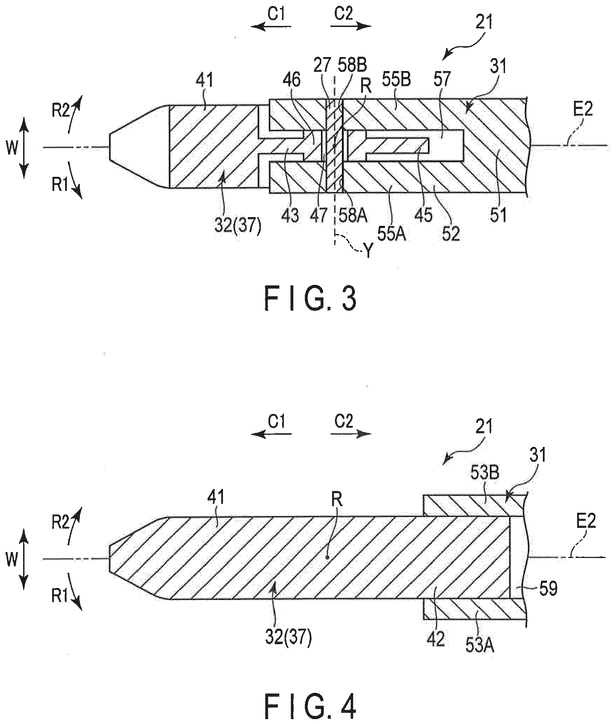

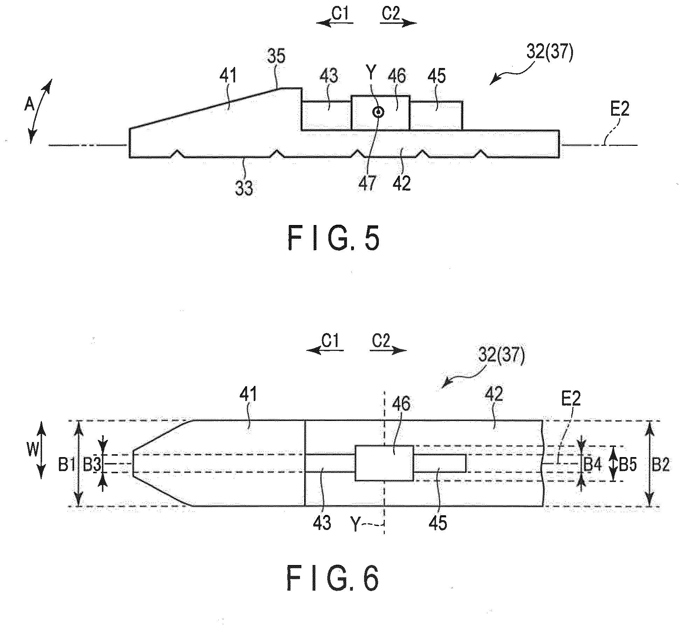

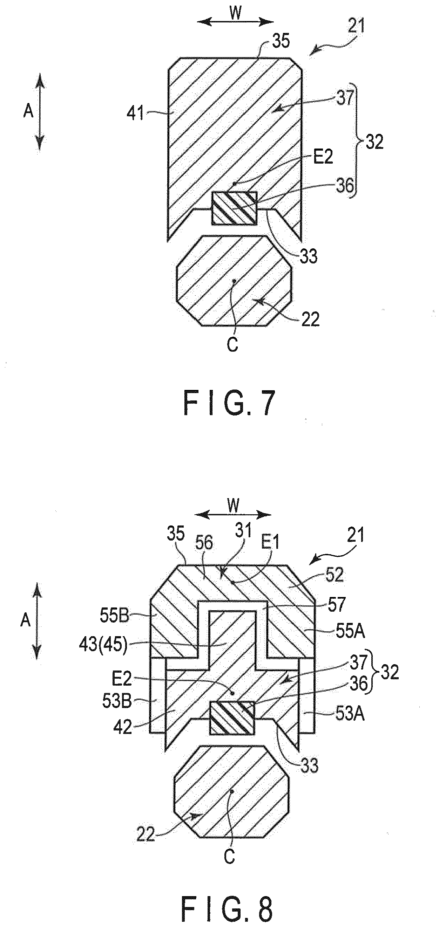

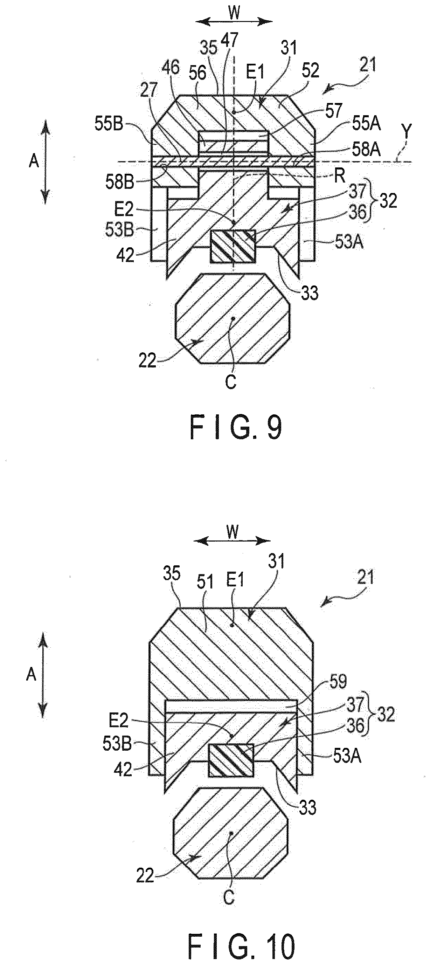

FIG. 3 is a cross-sectional view taken along X1-X1 in FIG. 2, and FIG. 4 is a cross-sectional view taken along X2-X2 in FIG. 2. FIG. 5 shows the swinger 32 viewed from one side in the direction along the swing axis Y (the width direction of the end effector 20), and FIG. 6 shows the swinger 32 viewed from the back surface 35 side in the open and close direction of the first clamp 21. FIG. 7 is a cross-sectional view taken along X3-X3 in FIG. 2, FIG. 8 is a cross-sectional view taken along X4-X4 and X6-X6 in FIG. 2, FIG. 9 is a cross-sectional view taken along X5-X5 in FIG. 2, and FIG. 10 is a cross-sectional view taken along X7-X7 in FIG. 2. As shown in FIGS. 2 to 10, the swinger 32 includes a pad 36 made of resin such as PTFE, and a hard holding member 37 made of metal and the like. In a state where the space between the clamps 21 and 22 is closed, the pad 36 can abut on the second clamp 22. In a state where the pad 36 abuts on the second clamp 22, the holding member 37 is separated from the second clamp 22.

The holding member 37 includes a member main body 41, and a base 42 continuous to the proximal side of the member main body 41. The member main body 41 forms the distal end of the holding member 37, and forms a part of the opposed surface 33 and a part of the back surface 35 of the first clamp 21. The base 42 forms the proximal end of the holding member 37, and forms a part of the opposed surface 33 of the first clamp 21. The member main body 41 and the base 42 of the holding member 37 integrally form the opposed surface 33 without any seam or step. The member main body 41 and the base 42 are formed across the entire width (from the edge to the edge) of the holding member 37 in the width direction of the end effector 20 (the direction indicated by the arrow W in each of FIGS. 3, 4, and 6-10). The holding member 37 includes a distal side projection 43, a proximal side projection 45, and a coupling projection 46 that project from the base 42 toward the back surface 35 side in the open and close direction of the first clamp 21. In the present embodiment, the distal side projection 43 is continuous to the proximal side of the member main body 41, the coupling projection 46 is continuous to the proximal side of the distal side projection 43, and the proximal side projection 45 is continuous to the proximal side of the coupling projection 46.

The dimension B3 of the distal side projection 43, the dimension B4 of the proximal side projection 45, and the dimension B5 of the coupling projection 46 are smaller as compared to the dimension B1 of the member main body 41 and the dimension B2 of the base 42 in the width direction of the end effector 20 (the direction along the swing axis Y). Thus, the widths of the distal side projection 43, the proximal side projection 45 and the coupling projection 46 along the width direction W of the end effector 20 are smaller than the full width of the holding member 37 of the end effector 20. The dimension B3 of the distal side projection 43 and the dimension B4 of the proximal side projection 45 are smaller than the dimension B5 of the coupling projection 46 in the width direction of the end effector 20. In the coupling projection 46, a through-hole 47 is formed along the width direction of the end effector 20, and the supporting pin 27 defining the swing axis Y is inserted through the through-hole 47.

The supporting section 31 includes a supporting section main body 51, and a concave section 52 continuous to the distal side of the supporting section main body 51. The concave section 52 is concaved to the back surface 35 side from the opposed surface 33 side in the open and close direction of the first clamp 21, and includes side walls 55A and 55B and a bottom wall 56. By the concave section 52, a hollow 57 is extended toward the proximal side from the distal end of the supporting section 31. The distal side projection 43, the proximal side projection 45, and the coupling projection 46 of the swinger 32 are inserted into the hollow 57, and the distal side projection 43, the proximal side projection 45, and the coupling projection 46 engage with the concave section 52 in the hollow 57. In each of the side walls 55A and 55B of the concave section 52, a corresponding fixed hole (corresponding one of 58A and 58B) is formed. With the supporting pin 27 being inserted through the through-hole 47 of the swinger 32 (coupling projection 46), one end of the supporting pin 27 is fixed to the side wall 55A by the fixed hole 58A, and the other end of the supporting pin 27 is fixed to the side wall 55B by the fixed hole 58B in the width direction of the end effector 20. In this manner, the supporting pin 27 is fixed to the supporting section 31, and the swing axis Y is defined along the direction intersecting with the extending axis E2 of the swinger 32 and intersecting with the open and close direction of the first clamp 21 (the direction indicated by the arrow A in each of FIGS. 5 and 7-10). In the state where the swinger 32 is located at the neutral position (the state where the extending axis E2 of the swinger 32 is substantially parallel to the extending axis E1 of the supporting section 31), the distal side projection 43, the proximal side projection 45, and the coupling projection 46 have no contact with the bottom wall 56 of the concave section 52.

The supporting section 31 includes engaging pieces 53A and 53B that project toward the opposed surface 33 side from the supporting section main body 51 in the open and close direction of the first clamp 21. It is preferable that the engaging piece 53A is integrated with the side wall 55A of the concave section 52 and the engagement piece 53B is integrated with the side wall 55B of the concave section 52. The engaging pieces 53A and 53B are separated from each other and a hollow 59 is formed between the engaging pieces 53A and 53B in the width direction of the end effector 20. The proximal portion of the base 42 of the swinger 32 (holding member 37) is inserted into the hollow 59, and the proximal portion of the base 42 engages with the engaging pieces 53A and 53B in the hollow 59. In a state where the swinger 32 is located at the neutral position, the proximal portion of the base 42 has no contact with the supporting section main body 51.

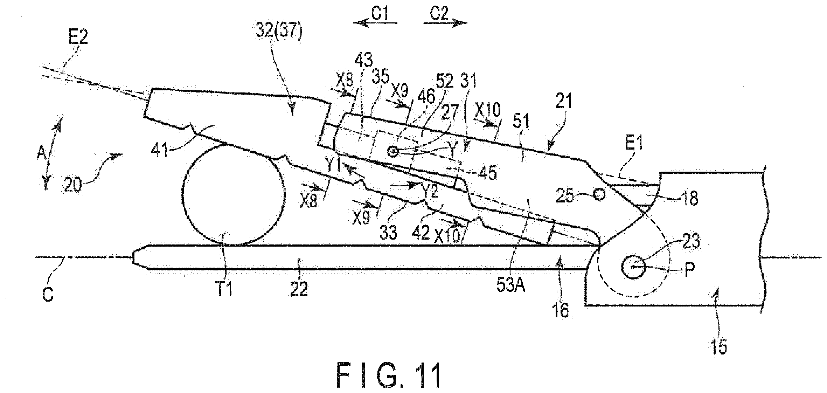

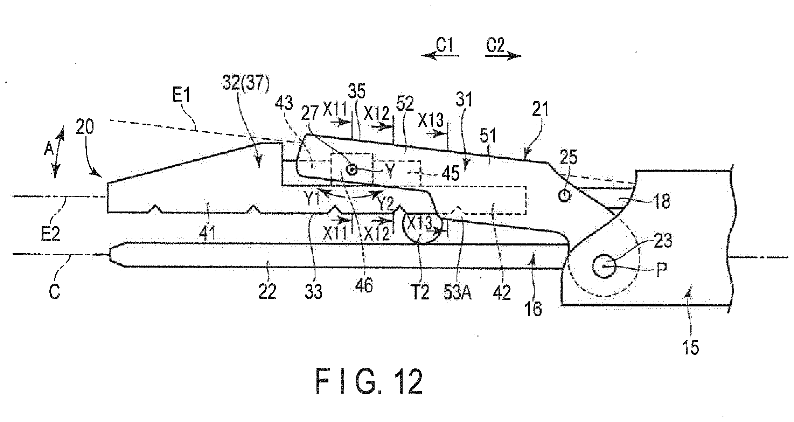

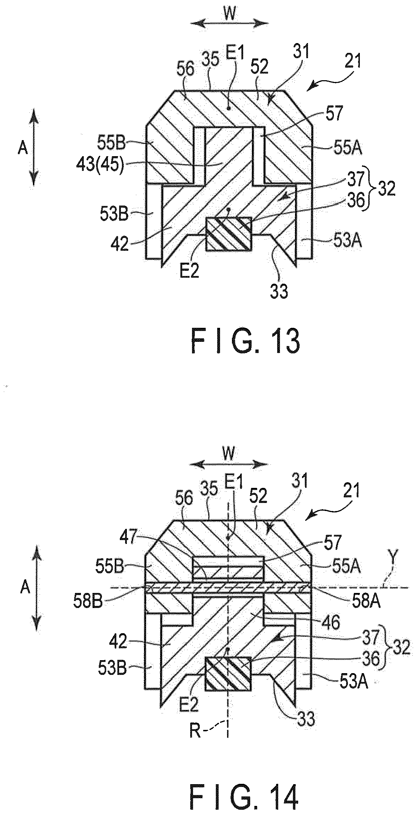

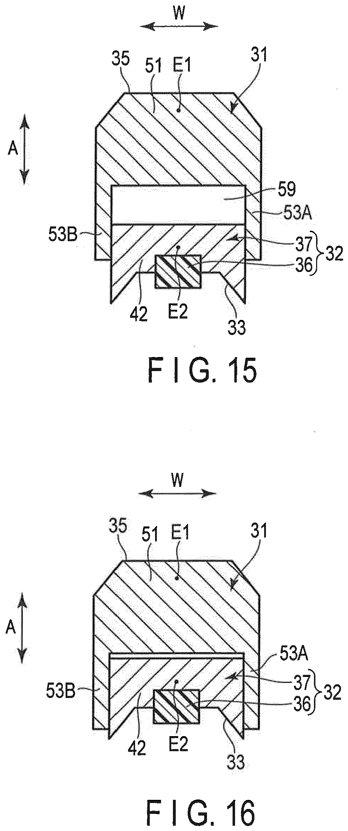

FIG. 11 shows the distal portion of the forceps-type treatment instrument 2 in a state where the swinger 32 is positioned where the proximal portion of the swinger 32 comes closest to the second clamp 22, and FIG. 12 shows the distal portion of the forceps-type treatment instrument 2 in a state where the swinger 32 is positioned where the distal portion of the swinger 32 comes closest to the second clamp 22. Each of FIGS. 11 and 12 shows the state viewed from one side in the width direction of the end effector 20. FIG. 13 is a cross-sectional view taken along X8-X8 in FIG. 11 and X12-X12 in FIG. 12, FIG. 14 is a cross-sectional view taken along X9-X9 in FIG. 11 and X11-X11 in FIG. 12, FIG. 15 is a cross-sectional view taken along X10-X10 in FIG. 11, and FIG. 16 is a cross-sectional view taken along X13-X13 in FIG. 12.

As shown in FIGS. 11 and 13, the swinger 32 is swingable to one side in the swing direction (the side indicated by the arrow Y1 in FIG. 11) until the distal side projection 43 (the projection end thereof) abuts on the bottom wall 56 of the concave section 52. In the state where the distal side projection 43 abuts on the bottom wall 56 of the concave section 52, the swinger 32 is positioned where the proximal portion of the swinger 32 (the portion at the proximal side relative to the swing axis Y in the swinger 32) comes closest to the second clamp 22. At this time, the bottom wall 56 regulates movement of the swinger 32 to one side in the swing direction. In the state where the swinger 32 is positioned where the proximal portion of the swinger 32 comes closest to the second clamp 22, the proximal side projection 45 and the coupling projection 46 have no contact with the bottom wall 56 of the concave section 52 (see FIG. 14), and the proximal portion of the base 42 has no contact with the supporting section main body 51 (see FIG. 15). When the swinger 32 swings to one side in the swing direction from a state where the swinger 32 is located at the neutral position (a state where the extending axis E2 of the swinger 32 is substantially parallel to the extending axis E1 of the supporting section 31), the extending axis E2 of the swinger 32 is inclined toward one side in the swing direction (the side indicated by the arrow Y1 in FIG. 11) with respect to the extending axis E1 of the supporting section 31.

As shown in FIGS. 12 and 13, on the other hand, the swinger 32 is swingable to the other side in the swing direction (the side indicated by the arrow Y2 in FIG. 12) until the proximal side projection 45 (the projection end thereof) abuts on the bottom wall 56 of the concave section 52. In the state where the proximal side projection 45 abuts on the bottom wall 56 of the concave section 52, the swinger 32 is positioned where the distal portion of the swinger 32 (the portion at the distal side relative to the swing axis Y in the swinger 32) comes closest to the second clamp 22. At this time, the bottom wall 56 regulates movement of the swinger 32 to the other side in the swing direction. In the state where the swinger 32 is positioned where the distal portion of the swinger 32 comes closest to the second clamp 22, the distal side projection 43 and the coupling projection 46 have no contact with the bottom wall 56 of the concave section 52 (see FIG. 14), and the proximal portion of the base 42 has no contact with the supporting section main body 51 (see FIG. 16). When the swinger 32 swings to the other side in the swing direction from a state where the swinger 32 is located at the neutral position (a state where the extending axis E2 of the swinger 32 is substantially parallel to the extending axis E1 of the supporting section 31), the extending axis E2 of the swinger 32 is inclined toward the other side in the swing direction (the side indicated by the arrow Y2 in FIG. 12) with respect to the extending axis E1 of the supporting section 31.

As described above, the swinger 32 swings between a position where the proximal portion of the swinger 32 (opposed surface 33) comes closest to the second clamp 22 and a position where the distal portion of the swinger 32 (opposed surface 33) comes closest to the second clamp 22. The swinging range of the swinger 32 includes the neutral position.

In the hollow 57, the side wall 55A of the concave section 52 abuts on the coupling projection 46 from one side in the width direction of the end effector 20, and the side wall 55B of the concave section 52 abuts on the coupling projection 46 from the other side in the width direction of the end effector 20. In each of the states where the swinger 32 is located at the neutral position, the swinger 32 is positioned where the proximal portion of the swinger 32 comes closest to the second clamp 22, and the swinger 32 is positioned where the distal portion of the swinger 32 comes closest to the second clamp 22, the side walls 55A and 55B of the concave section 52 of the supporting section 31 abut on the coupling projection 46 (see FIGS. 9 and 14). That is, even in a state where the swinger 32 is located at any position in the swinging range, the side walls 55A and 55B of the concave section 52 abut on the coupling projection 46. Thus, even in a state where the swinger 32 is located at any position in the swinging range, the coupling projection 46 is sandwiched between the side walls 55A and 55B in the direction along the swing axis Y (the width direction of the end effector 20), and at the coupling projection 46 (i.e., the portion through which the swing axis Y passes in the swinger 32), movement of the swinger 32 in the direction along the swing axis Y is regulated. Furthermore, even in a state where the swinger 32 is located at any position in the swinging range, the distal side projection 43 and the proximal side projection 45 are separated from the side walls 55A and 55B in the direction along the swing axis Y, and the distal side projection 43 and the proximal side projection 45 have no contact with the side walls 55A and 55B (see FIGS. 8 and 13) .

In the hollow 59, the engaging piece 53A abuts on the proximal portion of the base 42 from one side in the width direction of the end effector 20, and the engagement piece 53B abuts on the proximal portion of the base 42 from the other side in the width direction of the end effector 20. In each of the states where the swinger 32 is located at the neutral position, the swinger 32 is positioned where the proximal portion of the swinger 32 comes closest to the second clamp 22, and the swinger 32 is positioned where the distal portion of the swinger 32 comes closest to the second clamp 22, the engaging pieces 53A and 53B of the supporting section 31 abut on the proximal portion of the base 42 (see FIGS. 10, 15 and 16). That is, even in a state where the swinger 32 is located at any position in the swinging range, the engaging pieces 53A and 53B abut on the proximal portion of the base 42. Thus, even in a state where the swinger 32 is located at any position in the swinging range, the proximal portion of the base 42 is sandwiched between the engaging pieces 53A and 53B in the direction along the swing axis Y (the width direction of the end effector 20), and at the proximal portion of the base 42 (i.e., the proximal portion in the swinger 32), movement of the swinger 32 in the direction along the swing axis Y is regulated.

As described above, even in a state where the swinger 32 is located at any position in the swinging range, at the coupling projection 46 (the portion through which the swing axis Y passes in the swinger 32) and the proximal portion of the base 42 (the proximal portion in the swinger 32), movement of the swinger 32 in the direction along the swing axis Y is regulated. That is, the side walls 55A and 55B of the concave section 52 and the engaging pieces 53A and 53B form regulating sections. The regulating sections (53A, 53B, 55A, 55B), even in a state where the swinger 32 is located at any position in the swinging range, at two portions separated from each other in the direction along the extending axis E2 (the coupling projection 46 and the proximal portion of the base 42), regulate movement of the swinger 32 in the direction along the swing axis Y.

Next, the function and advantageous effects of the forceps-type treatment instrument 2 according to the present embodiment will be explained. When treatment is performed using the treatment system 1, the end effector 20 is inserted into a body cavity such as an abdominal cavity. Then, a treated target such as living tissue is placed between the clamps 21 and 22, and the handle 7 is closed with respect to the grip 6. The space between the clamps 21 and 22 is closed to grasp the treated target between the clamps 21 and 22. When an operator inputs the energy operation through the operation button 9, electronic energy is supplied from the energy control device 10 to the vibration generator 12, and ultrasonic vibration is generated by the vibration generator 12. The ultrasonic vibration generated by the vibration generator 12 is transmitted from the proximal side to the distal side through a vibration transmission member 16. The ultrasonic vibration is then transmitted to the second clamp 22, and the vibration transmission member 16 including the second clamp 22 vibrates. By vibration of the second clamp 22 with the treated target being grasped between the clamps 21 and 22, friction heat is generated between the second clamp 22 and the treated target, and the treated target is treated by the friction heat.

According to the present embodiment, in the first clamp 21, the swinger 32 is provided to be swingable around the swing axis Y with respect to the supporting section 31. Thus, as shown in FIG. 11, when the treatment target is grasped in a state where only the distal portion of the first clamp 21 (the distal portion of the opposed surface 33) and the distal portion of the second clamp 22 come in contact with a treated target T1, the swinger 32 swings to one side in the swing direction (the side indicated by the arrow Y1 in FIG. 11) from the state where it is located at the neutral position (the state where the extending axis E2 of the swinger 32 is substantially parallel to the extending axis E1 of the supporting section 31), and the proximal portion of the swinger 32 comes close to the second clamp 22. As the swinger 32 moves to one side in the swing direction, even if the treated target T1 is grasped in a state where only the distal portion of the first clamp 21 and the distal portion of the second clamp 22 have contact with the treated target T1, the treated target T1 is appropriately grasped with a grasp force more than a predetermined force.

As shown in FIG. 12, when the treated target T2 is grasped in a state where only the proximal portion of the first clamp 21 (proximal portion of the opposed surface 33) and the proximal portion of the second clamp 22 come in contact with the treated target T2, the swinger 32 swings to the other side in the swing direction (the side indicated by the arrow Y2 in FIG. 12) from the state where it is located at the neutral position, and the distal portion of the swinger 32 comes close to the second clamp 22. As the swinger 32 moves to the other side in the swing direction, even if the treated target T2 is grasped in a state where only the proximal portion of the first clamp 21 and the proximal portion of the second clamp 22 come into contact with the treated target, the treated target T2 is appropriately grasped with a grasp force more than a predetermined force.

A configuration in which the supporting section 31 is not provided with the engaging pieces 53A and 53B is exemplified as a comparative example. In this case, at the coupling projection 46, movement of the swinger 32 in the direction along the swing axis Y is regulated in the manner similar to the present embodiment, but unlike the present embodiment, at the proximal portion of the base 42, movement of the swinger 32 in the direction along the swing axis Y is not regulated. In the comparative example, at only one portion (the coupling projection 46), movement of the swinger 32 in the direction along the swing axis Y is regulated. Thus, when the swinger 32 moves in the direction along the swing axis Y at portions other than the coupling projection 46, the swinger 32 rotates (rattles) with respect to the supporting section 31 around the rotation axis R that is substantially parallel to the open and close direction of the first clamp 21 and passes through the coupling projection 46. That is, in the comparative example, there is a possibility that the swinger 32 rotates (rattles) in the rotation directions of the rotation axis R (the directions indicated by the arrows R1 and R2 in each of FIGS. 3 and 4). When the swinger 32 rotates around the rotation axis R, the angle of the extending axis E2 of the swinger 32 with respect to the direction along the swing axis Y changes.

In contrast, in the present embodiment, at the two portions separated from each other in the direction along the extending axis E2 of the swinger 32 (the coupling projection 46 and the proximal portion of the base 42), movement of the swinger 32 in the direction along the swing axis Y is regulated. Thus, the swinger 32 is prevented from rotating (rattling) around the rotation axis (along the open and close direction of the first clamp 21) substantially parallel to the open and close direction of the first clamp 21 (the direction indicated by the arrow A in FIG. 2), and for example, rotation (rattle) of the swinger 32 around the rotation axis R is prevented. It is therefore possible to suppress changes in the angle of the extending axis E2 of the swinger 32 (the extending direction of the swinger 32) with respect to the direction along the swing axis Y. In the present embodiment, the swinger 32 is prevented from rotating around the rotation axis (e.g., the rotation axis R) substantially parallel to the open and close direction of the first clamp 21, and therefore it is possible to suppress changes in the position of the swinger 32 with respect to the second clamp 22 in the width direction of the end effector 20 (the direction along the swing axis Y). Thus, in the present embodiment, the holding member 37 is prevented from coming in contact with the second clamp 22, and breakage of the second clamp 22 resulting from contact of the holding member 37 with the vibrating second clamp 22 can be effectively prevented.

In the present embodiment, moreover, even in a state where the swinger 32 is located at any position in the swinging range, at the coupling projection 46 (i.e., the portion through which the swing axis Y passes in the swinger 32) and the proximal portion of the base 42 (the proximal portion in the swinger 32), movement of the swinger 32 in the direction along the swing axis Y is regulated. Thus, at any positions in the swinging range of the swinger 32 (between the position where the distal portion of the swinger 32 comes closest to the second clamp 22 and the position where the proximal portion of the swinger 32 comes closest to the second clamp 22), the swinger 32 is prevented from rotating around the rotation axis substantially parallel to the open and close direction of the first clamp 21, and the position change of the swinger 32 with respect to the second clamp 22 in the width direction of the end effector 20 is prevented.

Moreover, in the present embodiment, the distal side projection 43 and the proximal side projection 45 have spaces with respect to the side walls 55A and 55B of the concave section 52 in the direction along the swing axis Y. Thus, in the swinger 32, members (including the supporting section 31) other than the swinger 32 abut only on the proximal portion of the base 42 and the coupling projection 46 from both sides in the direction along the swing axis Y. Thus, even if the swinger 32 swings to the supporting section 31, no friction is generated at portions other than the proximal portion of the base 42 and the coupling projection 46 in the swinger 32. It is thus possible to reduce adverse effects of the friction on the swing of the swinger 32.

### (Modifications)

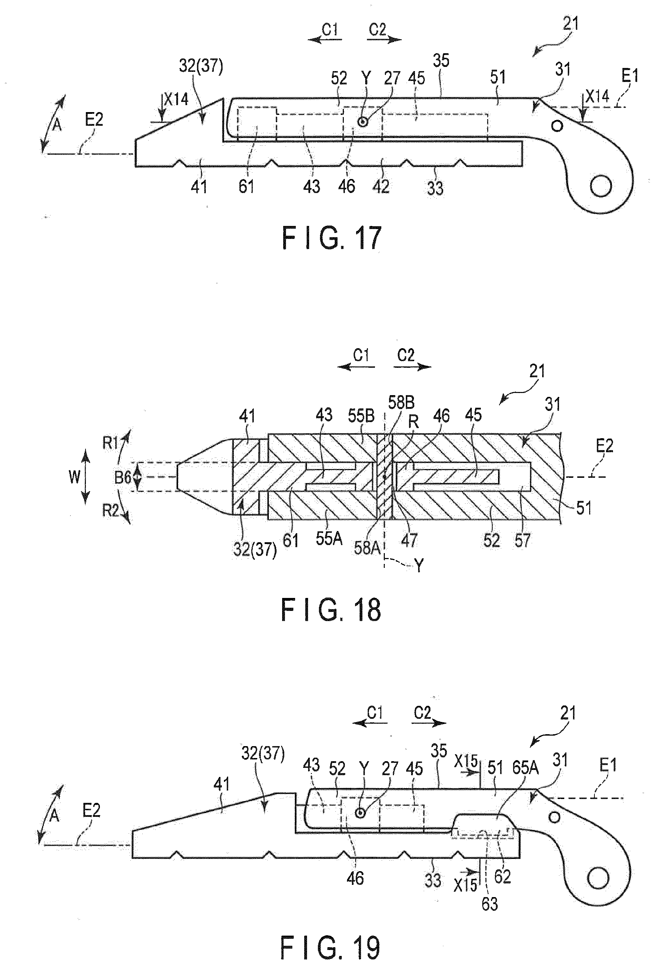

In the first embodiment, at the coupling projection (the portion through which the swing axis Y passes) and the proximal portion of the base 42 (the proximal portion of the swinger 32), movement of the swinger 32 in the direction along the swing axis Y is regulated, however, it is not limited to this. For example, in the first modification shown in FIGS. 17 and 18, the supporting section 31 is not provided with the engaging pieces 53A and 53B. Instead, in this modification, the holding member 37 of the swinger 32 is provided with an abutment projection 61. FIG. 17 shows the first clamp 21 viewed from one side in the width direction of the end effector 20, and FIG. 18 is a cross-sectional view taken along X14-X14 in FIG. 17.

As shown in FIGS. 17 and 18, the abutment projection 61 is provided at the distal portion of the swinger 32, and projects from the base 42 toward the back surface 35 side in the open and close direction of the first clamp 21. The abutment projection 61 is continuous between the member main body 41 and the distal side projection 43 in the direction along the extending axis E2. The dimension B6 of the abutment projection 61 is larger than the dimension B3 of the distal side projection 43 and the dimension B4 of the proximal side projection 45 and substantially the same as the dimension B5 of the coupling projection 46 in the width direction of the end effector 20 (the direction along the swing axis Y). In this modification, the abutment projection 61 in addition to the distal side projection 43, the proximal side projection 45, and the coupling projection 46 are inserted into the hollow 57 formed by the concave section 52, and the abutment projection 61 in addition to the distal side projection 43, the proximal side projection 45 and the coupling projection 46 engage with the concave section 52 of the supporting section 31.

In this modification, in the hollow 57, the side wall 55A of the concave section 52 abuts on the abutment projection 61 from one side in the width direction of the end effector 20, and the side wall 55B of the concave section 52 abuts on the abutment projection 61 from the other side in the width direction of the end effector 20. Moreover, in this modification, even in a state where the swinger 32 is located at any position in the swinging range, the side walls 55A and 55B of the concave section 52 abut on the abutment projection 61. Thus, even in a state where the swinger 32 is located at any position in the swinging range, the abutment projection 61 is sandwiched between the side walls 55A and 55B in the direction along the swing axis Y (the width direction of the end effector 20), and at the abutment projection 61 (i.e., the distal portion of the swinger 32), movement of the swinger 32 in the direction along the swing axis Y is regulated. In this modification, moreover, even in a state where the swinger 32 is located at any position in the swinging range, at the coupling projection 46 (i.e., the portion through which the swing axis Y passes in the swinger 32), movement of the swinger 32 in the direction along the swing axis Y is regulated. Furthermore, in this modification, even in a state where the swinger 32 is located at any position in the swinging range, the distal side projection 43 and the proximal side projection 45 in the direction along the swing axis Y are separated from the side walls 55A and 55B, and the distal side projection 43 and the proximal side projection 45 have no contact with the side walls 55A and 55B.

With the configuration as described above, in this modification, even in a state where the swinger 32 is located at any position in the swinging range, at the coupling projection 46 (the portion through which the swing axis Y passes in the swinger 32) and the abutment projection 61 (the distal portion of the swinger 32), movement of the swinger 32 in the direction along the swing axis Y is regulated. That is, the side walls 55A and 55B of the concave section 52 form regulating sections. The regulating sections (55A, 55B), even in a state where the swinger 32 is located at any position in the swinging range, at two portions separated from each other in the direction along the extending axis E2 (the coupling projection 46 and the abutment projection 61), regulate movement of the swinger 32 in the direction along the swing axis Y. Thus, also in this modification, the swinger 32 is prevented from rotating (rattling) around the rotation axis substantially parallel to the open and close direction of the first clamp 21 (the direction indicated by the arrow A in FIG. 17), and for example, rotation of the swinger 32 around the rotation axis R (the directions indicated by the arrows R1 and R2 in the FIG. 18) is prevented.

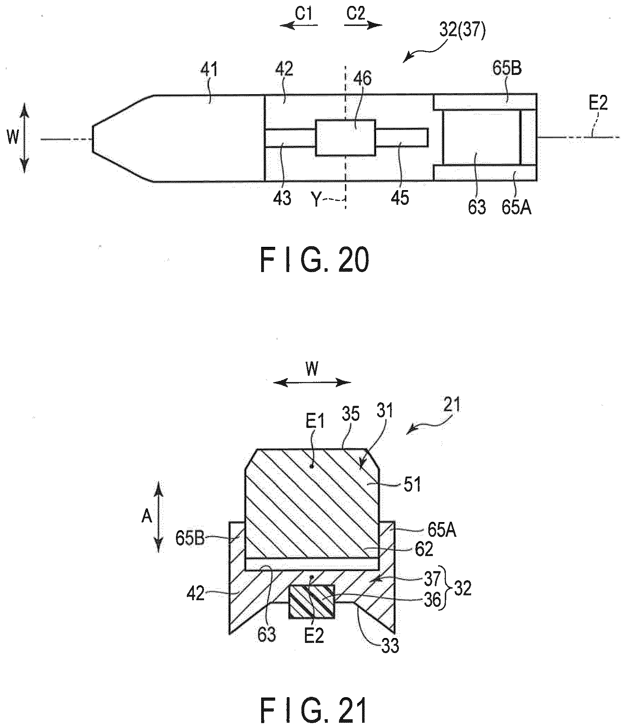

In a second modification shown in FIGS. 19 to 21, the supporting section 31 is not provided with the engaging pieces 53A and 53B, but the holding member 37 (swinger 32) is provided with engaging pieces 65A and 65B instead. FIG. 19 shows the first clamp 21 viewed from one side in the width direction of the end effector 20, and FIG. 20 shows the swinger 32 (holding member 37) viewed from the back surface 35 side in the open and close direction of the first clamp 21. Also, the cross section taken along X15-X15 in FIG. 21 is shown.

As shown in FIGS. 19 to 21, in the holding member 37, the engagement pieces 65A and 65B project from the proximal portion of the base 42 toward the back surface 35 side in the open and close direction of the first clamp 21. The engagement pieces 65A and 65B are separated from each other in the width direction of the end effector 20 (the direction along the swing axis Y). In the proximal portion of the base 42, a concave section 63 that is concaved toward the opposed surface 33 side in the open and close direction of the first clamp 21 is formed. The concave section 63 is positioned between the engagement pieces 65A and 65B in the width direction of the end effector 20, and is positioned at the proximal side with respect to the proximal side projection 45. In this modification, the supporting section 31 is provided with a supporting section projection 62 that projects from the supporting section main body 51 toward the concave section 63. The supporting section projection 62 projects from the supporting section main body 51 toward the opposed surface 33 side in the open and close direction of the first clamp 21. In this modification, the supporting section projection 62 is positioned at the proximal side with respect to the proximal side projection 45 of the swinger 32, and is inserted into the concave section 63 of the swinger 32.

In this modification, in the concave section 63 of the swinger 32 (holding member 37), the supporting section projection 62 of the supporting section 31 abuts on the engaging piece 65A from one side in the width direction of the end effector 20, and the supporting section projection 62 abuts on the engaging piece 65B from the other side in the width direction of the end effector 20. Moreover, in this modification, even in a state where the swinger 32 is located at any position in the swinging range, the supporting section projection 62 abuts on the engaging pieces 65A and 65B. Thus, even in a state where the swinger 32 is located at any position in the swinging range, the supporting section projection 62 is sandwiched between the engaging pieces 65A and 65B in the direction along the swing axis Y (the width direction of the end effector 20), and at the engaging pieces 65A and 65B (i.e., the distal portion of the swinger 32), movement of the swinger 32 in the direction along the swing axis Y is regulated. In this modification, moreover, even in a state where the swinger 32 is located at any position in the swinging range, at the coupling projection 46 (i.e., the portion through which the swing axis Y passes in the swinger 32), movement of the swinger 32 in the direction along the swing axis Y is regulated.

With the configuration as described above, in this modification, even in a state where the swinger 32 is located at any position in the swinging range, at the coupling projection 46 (the portion through which the swing axis Y passes in the swinger 32) and the engaging pieces 65A and 65B (the proximal portion of the swinger 32), movement of the swinger 32 in the direction along the swing axis Y is regulated. That is, the side walls 55A and 55B of the concave section 52 and the supporting section projection 62 form regulating sections. The regulating sections (55A, 55B, 62), even in a state where the swinger 32 is located at any position in the swinging range, at two portions separated from each other in the direction along the extending axis E2 (the coupling projection 46 and the engaging pieces 65A and 65B), regulate movement of the swinger 32 in the direction along the swing axis Y. Thus, in this modification, the swinger 32 is prevented from rotating (rattling) around the rotation axis (e.g., rotation axis R) substantially parallel to the open and close direction of the first clamp 21 (the direction indicated by the arrow A in FIG. 19).

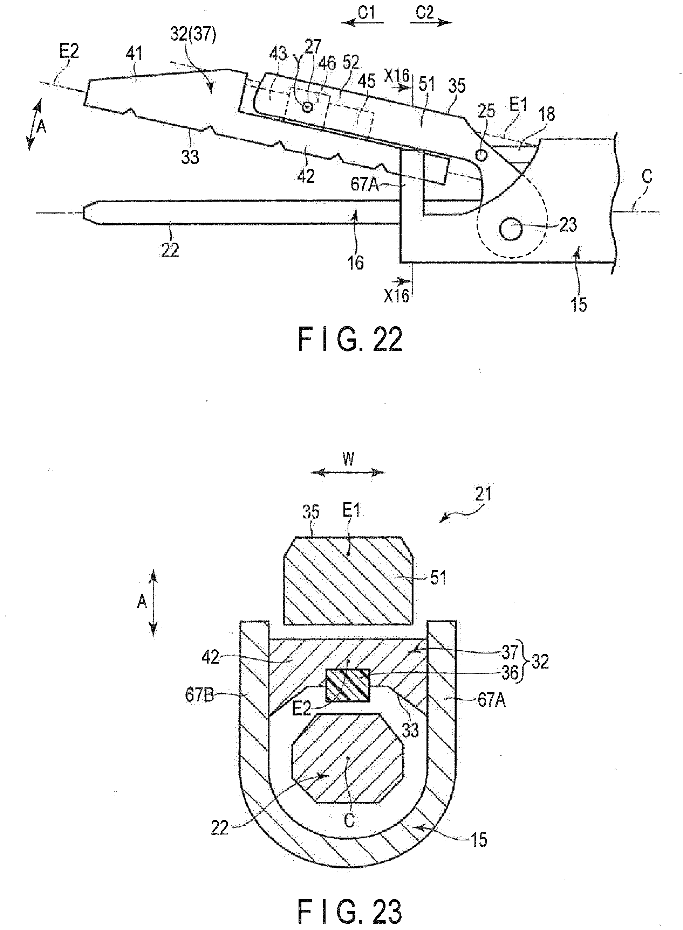

In a third modification shown in FIGS. 22 and 23, the supporting section 31 is not provided with the engaging pieces 53A and 53B, but the distal portion of the sheath 15 is provided with engaging pieces 67A and 67B instead. FIG. 22 shows the distal portion of the forceps-type treatment instrument 2 viewed from one side in the width direction of the end effector 20, and FIG. 23 is a cross-sectional view taken along X16-X16 in FIG. 22.

As shown in FIGS. 22 and 23, in the distal portion of the sheath 15, the engaging pieces 67A and 67B project toward the first clamp 21 side from the second clamp 22 side. The engagement pieces 67A and 67B are separated from each other in the width direction of the end effector 20 (the direction along the swing axis Y). In this modification, the proximal portion of the base 42 of the swinger 32 is inserted between the engaging pieces 67A and 67B.

In this modification, between the engaging pieces 67A and 67B, the engaging piece 67A abuts on the proximal portion of the base 42 from one side in the width direction of the end effector 20, and the engaging piece 67B abuts on the proximal portion of the base 42 from the other side in the width direction of the end effector 20. In this modification, in a state where the swinger 32 is located at any position in the swinging range, the engaging pieces 67A and 67B abut on the proximal portion of the base 42 of the swinger 32. Thus, even in a state where the swinger 32 is located at any position in the swinging range, the proximal portion of the base 42 is sandwiched between the engaging pieces 67A and 67B in the direction along the swing axis Y (the width direction of the end effector 20), and at the proximal portion of the base 42 (i.e., the proximal portion of the swinger 32), movement of the swinger 32 in the direction along the swing axis Y is regulated. In this modification, moreover, even in a state where the swinger 32 is located at any position in the swinging range, at the coupling projection 46 (i.e., the portion through which the swing axis Y passes in the swinger 32), movement of the swinger 32 in the direction along the swing axis Y is regulated.

With the configuration as described above, in this modification, even in a state where the swinger 32 is located at any position in the swinging range, at the coupling projection 46 (the portion through which the swing axis Y passes in the swinger 32) and the proximal portion of the base 42 (the proximal portion of the swinger 32), movement of the swinger 32 in the direction along the swing axis Y is regulated. That is, the side walls 55A and 55B of the concave section 52 and the engaging pieces 67A and 67B of the sheath 15 form regulating sections. The regulating sections (55A, 55B, 67A, 67B), even in a state where the swinger 32 is located at any position in the swinging range, at two portions separated from each other in the direction along the extending axis E2 (the coupling projection 46 and the proximal portion of the base portion 42), regulate movement of the swinger 32 in the direction along the swing axis Y. Thus, in this modification, the swinger 32 is prevented from rotating (rattling) around the rotation axis (e.g., rotation axis R) substantially parallel to the open and close direction of the first clamp 21 (the direction indicated by the arrow A in FIG. 22).

In the embodiment and the like described above, at the two portions separated from each other in the direction along the extending axis E2, movement of the swinger 32 in the direction along the swing axis Y is regulated, however, it is not limited to this. In one modification, at three or more portions separated from one another in the direction along the extending axis E2, movement of the swinger 32 in the direction along the swing axis Y may be regulated. In this case, for example, at the portion through which the swing axis Y passes in the swinger 32, the distal portion of the swinger 32, and the proximal portion of the swinger 32, movement of the swinger 32 in the direction along the swing axis Y is regulated. That is, it is only required that in at least two portions separated from each other in the direction along the extending axis E2, movement of the swinger 32 in the direction along the swing axis Y is regulated.

Furthermore, in one modification, instead of electric energy (vibration generation electric energy) supplied to the vibration generator 12 or in addition to the electric energy supplied to the vibration generator 12, high-frequency electric energy may be output from the energy control device 10 to supply high-frequency electric energy to the holding member 37 of the first clamp 21 and the second clamp 22. In this case, when the high-frequency electric energy is supplied to the holding member 37 and the second clamp 22, a high-frequency current is applied to the grasped treated target, and treatment is performed using the high-frequency current. In one modification, moreover, ultrasonic vibration and energies such as high-frequency electric energy may not be transmitted to the end effector 20.

The configuration in which the space between the clamps 21 and 22 is opened or closed is not limited to the configuration of the above-described embodiment and the like. For example, in one modification, a handle (7) and a first clamp (21) may be rotatable together with respect to a sheath (15) and a housing (3). In this case, by opening or closing the handle (7) with respect to the housing (3), the first clamp (21) rotates integral with the handle (7), and the space between a pair of clamps (21 and 22) is opened and closed. In a different modification, both clamps (21 and 22) may be rotatable with respect to the sheath 15. In this case, by opening and closing the handle (7) with respect to a grip (6), a movable body (18) moves along a longitudinal axis (C) and both clamps (21 and 22) rotate with respect to the sheath (15). In this manner, a space between the clamps (21 and 22) is opened and closed.

In the embodiment and the like described above, the forceps-type treatment instrument (2) includes an end effector (20), and the end effector (20) includes the first clamp (21), and the second clap (22) that is openable and closable relative to the first clamp (21). The first clamp (21) includes a supporting section (31) and a swinger (32), and the swinger (32) is provided opposed to the second clamp (22) and extended along an extending axis (E2) from the distal portion to the proximal portion. The swinger (32) swings relative to the supporting section (31) around the swing axis (Y) that is along the direction intersecting with the extending axis (E2) and intersecting with the open and close direction (A) of the first clamp (21), and swings in a swinging range between a position where the distal portion comes closest to the second clamp (22) and a position where the proximal portion comes closest to the second clamp (22). Even in a state where the swinger (32) is located at any position in the swinging range, the regulating section (53A, 53B, 55A, 55B; 55A, 55B; 55A, 55B, 62; 55A, 55B, 67A, 67B), in at least two positions separated from each other in the direction along the extending axis (E2), regulates movement of the swinger (32) in the direction along the swing axis (Y), and the swinger (32) is prevented from rotating around the rotation axis (R) along the open and close direction (A) of the first clamp (21).

Although the embodiments, etc. of the present invention have been described above, the present invention is not limited to the above-described embodiments, etc., and, needless to say, various modifications can be made without departing from the spirit of the invention.

## Claims

1. A forceps-type treatment instrument, comprising:
an end effector including a first clamp, and a second clamp that is openable and closable relative to the first clamp;
a supporting section provided in the first clamp;
a swinger provided in the first clamp to be opposed to the second clamp, and extended along an extending axis from a distal portion to a proximal portion, the swinger swinging with respect to the supporting section around a swing axis, which is extended along a direction intersecting with the extending axis and intersecting with an open and close direction of the first clamp, the swinger swinging in a swinging range between a position where the distal portion comes closest to the second clamp and a position where the proximal portion comes closest to the second clamp; and
a regulating section regulating movement of the swinger in the direction along the swing axis in at least two portions separated from each other in a direction along the extending axis, and thereby preventing rotation of the swinger around a rotation axis along the open and close direction of the first clamp, in a state where the swinger is located at any position in the swinging range.

2. The forceps-type treatment instrument according to claim 1,
wherein the regulating section regulates movement of the swinger in the direction along the swing axis in at least one of the proximal portion and the distal portion of the swinger.

3. The forceps-type treatment instrument according to claim 1,
wherein the regulating section regulates movement of the swinger in the direction along the swing axis in a portion through which the swing axis passes.

4. The forceps-type treatment instrument according to claim 1,
wherein the supporting section includes a concave section concaved toward a side away from the second clamp in the open and close direction of the first clamp, the swinger engaging with the concave section, and
the regulating section is provided in the concave section and regulates movement of the swinger in the direction along the swing axis by abutment of the swinger.
